Europäisches Patentamt

European Patent Office

Office européen des brevets

(19)

(11) Publication number: **0 275 811**
**A1**

# (12) EUROPEAN PATENT APPLICATION

(21) Application number: **87600015.9**

(22) Date of filing: **17.12.87**

(51) Int. Cl.⁴: **A61B 5/04**

(30) Priority: **22.12.86 US 946519**
**06.11.87 US 118279**

(43) Date of publication of application:
**27.07.88 Bulletin 88/30**

(84) Designated Contracting States:
**AT BE CH DE FR GB IT LI LU NL SE**

(71) Applicant: **CARDIOTRONICS INTERNATIONAL, INC.**
**1840 Century Park East**
**Los Angeles, CA 90067(US)**

(72) Inventor: **Lavine, Thomas G**
**2496 Angelo Drive**
**Los Angeles, CA(US)**

(74) Representative: **Papaharalabus, Catherine C.**
**P.O. Box 8181**
**GR-100 10 Athens(GR)**

(54) **EKG sensor sheet.**

(57) A device for the acquisition of electrocardiogram signals includes a flexible sheet (10) which incorporates a cable connector (30), multiple lead wires (20-29) and a plurality of electrical contacts (36) prepositioned for placement on a patient's chest. An embodiment includes lead wires and contacts prepositioned for a standard full screen, 12-lead electrocardiogram or EKG.

FIG. 3

FIG. 4

EP 0 275 811 A1

## EKG SENSOR SHEET

This application is a continuation-in-part of application Serial No. 946,519, filed December 22, 1986 of T.G. Lavine.

## Field of the Invention

This invention relates to devices for electrocardiographic monitoring and telemetry, and more particularly devices for interfacing between a patient and electrocardiography apparatus.

## Background of the Invention

Electrocardiographic monitoring and telemetry have been in use in the United States for some time for screening and diagnosis of cardiopathy. Electrocardiography provides a graphic registration, commonly known as an EKG, of movements of the heart using electrical signal sensing. In general, electrical leads used to transmit signals from the patient's cardiovascular system are fixed to the skin of the patient individually, at suitable locations, using suction cups and conductive adhesives to provide the necessary electrical connection for reading the heart movements through a patient's skin.

For screening and evaluation, the patient should be ambulatory so that readings can be taken during different degrees of physical exertion to demonstrate the reaction of the heart to differing stresses. A standard type of testing includes electrocardiographic monitoring of a patient during a set of standardized treadmill exercises. It is well recognized that this type of screening and evaluation are important for diagnosis of new ischemic cardiac episodes in the chronically symptomatic cardiac patient as well as the identification of "silent ischemic" periods of myocardial oxygen insufficiency not associated with classic chest-pain. These ischemic electrocardiographic changes can be accurately recognized only if a full screen electrocardiogram is performed. The standard full screen electrocardiogram includes readings taken by electrically conductive leads at standard chest positions combined to provide readings.

Periodic electrocardiograms can provide a physician with a cardiographic profile of an individual patient for early detection and diagnosis of cardiovascular diseases. For purposes of providing an accurate profile, it is important that each electrocardiogram be taken with leads affixed about same location on the patient. The accuracy of each electrocardiogram requires that the leads be accurately placed and that they remain fixed while the patient is ambulatory. In addition, to provide accurate information for an individual patient, a series of electrocardiograms are taken and compared to one another to detect changes in the reaction of the individual patient's heart to the same stresses. Therefore, it is important that placement of the leads be consistent from one electrocardiogram session to another.

As can be appreciated, accurately placing and securing a large number of leads can be difficult and time consuming. In addition, electrocardiograms are taken periodically and the results compared to one another to provide a continuing profile of an individual patient's heart movements for early diagnosis and treatment of heart disease and to identify "silent ischemic" periods of myocardial oxygen insufficiency in chronically symptomatic cardiac patients. It would therefore be advantageous to have a device for accurate placement of leads for accurately reproducing test conditions for comparison between testing episodes.

Although a full screen, twelve point electrocardiogram provides the most accurate picture for recognizing ischemic electrocardiographic changes, because of the time required to place and secure individual leads, electrocardiograms taken during an acute symptomatic episode of a cardiac patient are generally limited to two-to four-lead readings. It would therefore be advantageous to have a device which enables more leads to be accurately placed and secured quickly during an acute symptomatic episode.

U.S. Patent No. 4,608,987 to H.E. Mills relates to a vest-like garment having a plurality of aperatures adapted for receiving electrodes. However, there is no provision to ensure that the electrodes are placed each time of reuse of the vest about the same location.

U.S. Patent No. 4,583,549 to S. Manoli relates to an ECG electrode pad with a plurality of ECG electrodes which are repositioned with regard to each other and not with reference to their previous position.

U.S. Patent No. 4,365,634 to Bare et al discloses a multi-terminal electrode construction having a pair of separate support members adapted for the transcutaneous application of an electrode to a patient. A multiterminal design is provided by a conductive pattern printed on a semiflexible sheet. The pattern is printed with conductive ink in a binder composition. However, there is no means provided which can ensure the reapplication of the terminals to the same location so as to obtain a better comparison of test results.

U.S. Patent No. 4,593,698 to R.J. Athans discloses an electrocardiograph sensor positioning device for repeatedly positioning electrocardiograph sensors. The device establishes a longitudinal reference between two anatomical landmarks on an individual. A second reference path is then found and recorded. The recorded locations are necessary to ensure a similar location. One of the problems with the use of the device requires that the second examining physician have the information from the first physician in order to obtain readings at the same locations. The device of the invention provides uniform examination without requiring information from others on placement of the electrodes.

Telephonic units for transmitting ECG signals to ECG receiving equipment at a cardiologist's office are described generally in U.S. Patent No. 3,910,260. Usually such transmission takes place in emergency vehicles where prior medical history may not be readily available. In order to obtain meaningful and reliable information repeatable ECG signals are necessary for the cardiologist. None of the prior art devices have provided a solution to obtaining repeatable ECG signals in the field by untrained or non-professional parties.

The present invention provides accurate, repeatable placement of leads for electrocardiograms which is repeatable without knowlege of the previous locations. The device of this invention lowers the time involved in placement and affixation by providing a sensor sheet incorporating multiple leads which are preferably pre-wired to a terminus that can connect to a standard electrocardiographic cable or to a telemetric unit, as more fully discussed below.

## SUMMARY OF THE INVENTION

In general, the present invention includes a flexible sheet or bib for electrically connecting a patient with an electrocardiographic monitoring device. The bib has incorporated therein electrical conducting means, e.g. wires, for transmitting electrical impulses from a patient to be monitored to the electrocardiograph device. The bib incorporates at least six, preferably 8-12, electrical conductors or electrodes in a predetermined pattern corresponding to a standard, full screen electrocardiogram.

Each of the conductors or electrodes includes a first end mounted for connection to predetermined positions on a patient's chest and a second end for connection with an electrocardiographic device.

More particularly, the present invention includes a non-conductive substrate, for example a sheet of non-conductive natural or plastic material for example polyvinyl chloride, polyethylene, polyphenylene, terephthalate, cotton, rayon, and the like, having incorporated therein a plurality of electrically conductive wires or leads. Each lead includes a first end portion or receptor adapted for electrical connection with the skin of the patient for receiving electrical impulses. A second end of each lead terminates in a common electrical connector or cable junction preferably adapted for connection with a standard type of cable juncture for connection with the electrocardiograph device.

It is a feature of the invention to provide electrically conductive adhesive means pre-applied to the conductors or electrodes for assuring electrical contact with the skin. One or more positioning members are included to facilitate positioning the bib in the proper location on the patient's chest to assure that each of the prepositioned conductors or electrodes is located in appropriate overlying relationship for obtaining readings at selected locations on the patient's chest. The positioning members are advantageously located to overlie the easily recognizable bony chest landmarks of the patient, i.e. the clavicles and sternum. The positioning members are slots, notches, openings in the bib for locating by touch the sternum notch and the clavicle notch.

A standard pattern may be printed in any suitable manner on the bib to further facilitate positioning of the bib on the patient. The pattern includes an outline of the clavicles and sternum landmarks, or any other recognizable outline of chest landmarks.

It is therefore an object of the present invention to provide a method and apparatus which may repeatedly position a plurality of body electrodes about the same position on the body without knowledge of the prior positioning.

It is a further object of the invention to provide a garment for supporting a plurality of electrocardiograph electrodes which are set in a predetermined pattern that can be repeatedly placed on a patient's body about the same position for connection to electrocardiograph equipment.

It is a still further object of the invention to provide a garment having a plurality of electrocardiograph electrodes which can be placed on a patient by untrained parties or the patient himself which can provide reliable and repeatable ECG signals.

## BRIEF DESCRIPTION OF THE DRAWINGS

Figure 1 is a front view of a preferred embodiment of the invention.

Figure 2 is an enlarged, cross sectional view taken along line II-II of Figure 1.

Figure 3 is a view similar to Figure 1, showing the preferred embodiment in phantom to illustrate placement of the sheet on a patient.

Figure 4 is similar to Figure 3 and illustrates the sternum and clavicle positioning means.

## DETAILED DESCRIPTION OF THE PREFERRED EMBODIMENTS

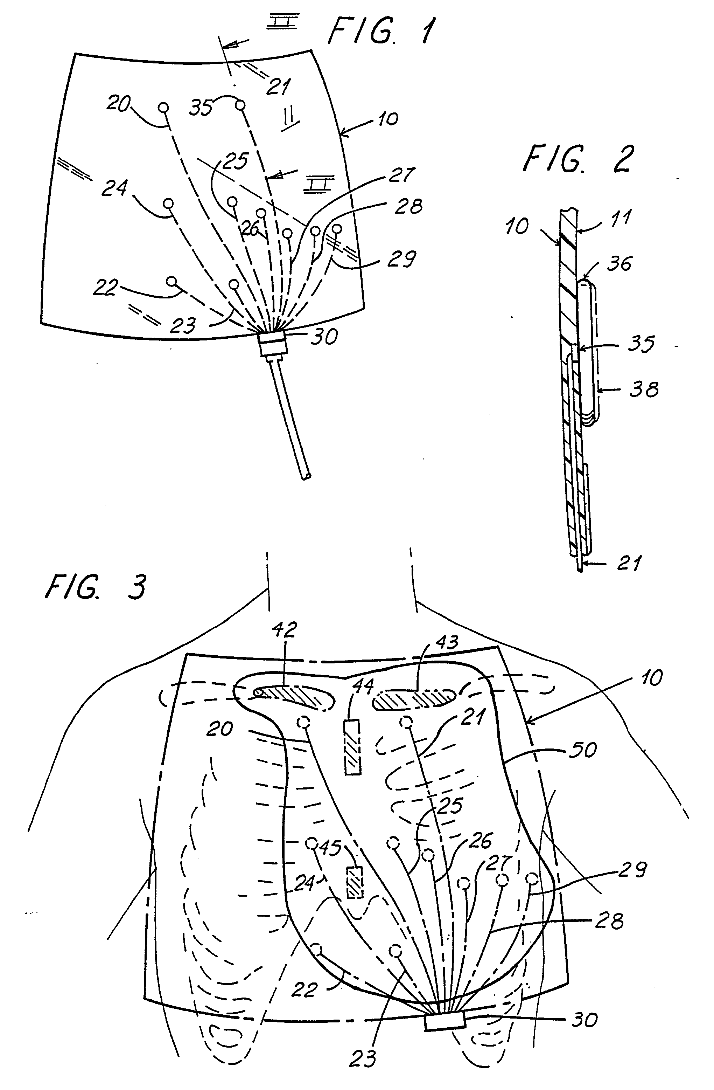

As illustrated in Figure 1, a preferred embodiment of the EKG sensor sheet of the instant invention includes flexible sheet or bib 10 having a contact surface 11 for placement on the chest region of a patient. The bib 10 is preferably sized to cover the entire chest area of a patient as illustrated in Figure 3.

A plurality of electrically conductive leads are incorporated into the bib 10 to transmit electrical impulses from the patient's chest to an electrocardiographic recording device. In the preferred embodiment illustrated in the Drawing, with reference to Figure 1, the leads are configured in the pattern normally used in the chest configuration for standard treadmill exercise testing. More particularly, the leads include the normal limb leads for extremities such as a right arm lead 20, left arm lead 21, right leg lead 22, left leg lead 23 as well as the normal chest leads 24, 25, 26, 27, 28 and 29. The four limb leads alone can provide a standard two-or four-lead electrocardiogram. The four limb leads when combined with the chest leads 24 and 25 in addition to the individual leads provide the standard 12 lead electrocardiogram.

With reference to Figure 3, the right arm lead 20 and the left arm lead 21 are located below the clavicles bilaterally. The right leg lead 22 and left leg lead 23 are located below the xyphoid process of the sternum. The chest lead 26 is located approximately at the midclavicle, and the chest lead 29 is located approximately at the mid-axillary line. Each of the leads 20 - 29 is electrically connected at one end to a suitable connecter such as cable junction 30 illustrated in Figures 1 and 3.

With reference to Figure 2, each of the leads 20-29 includes a receptor end, such as receptor 35 of the left arm lead 21. The receptor 35 may include any suitable electrical contact 36 such as copper, gold plate or conductive plastic, mounted to the lead 21 and exposed on the contact surface 11 of the bib 10 for contact with the patient's skin. A conductive adhesive or gel 38 is preferably applied to the receptor 35 to assure good electrical contact with the patient's skin.

Although the individual receptors, such as the receptor 35 having the conductive adhesive 38 may provide sufficient adhesion for maintaining the bib 10 in position, it is an additional feature of the present invention to provide two or more positioning members for more accurate placement of the bib on a patient's chest. In a preferred embodiment as illustrated in Figure 3, the positioning members are located for placement over bony portions of the anatomy which are easily recognized. More particularly, in the illustrated embodiment the positioning members are located for placement over the clavicles and sternum, the bony chest landmarks, and include clavicle patches 42 and 43, an upper sternum patch 44, and a lower sternum patch 45. The positioning members or patches 42 - 45 may include any suitable adhesive applied to the contact surface 11 of the bib 10.

As can now be appreciated, by mounting the leads 20 - 29 securely to the bib 10 and thus prepositioning the receptors such as the receptor 35, accurate placement for accurate and reproducible test results is greatly facilitated. In addition, the possibility of receptors being dislodged by leads becoming entangled with one another or the surroundings during treadmill exercises is greatly reduced. The positioning patches 42 - 45 further assure that the bib 10 will remain in place throughout the testing session.

For additional ease in placement, the bib 10 may be color-encoded on the visible or non-contacting bib surface for ready recognition of the bony, chest landmarks, and may include, for example a colored portion on the non-contacting surface of the bib 10 as illustrated by hatching shown on the patches 42 - 45 in Figure 3. An outline and/or colored markings for each of the clavicles and sternum and/or lead receptors such as outline 50 may also be printed on the non-contacting bib surface for ease in bib placement.

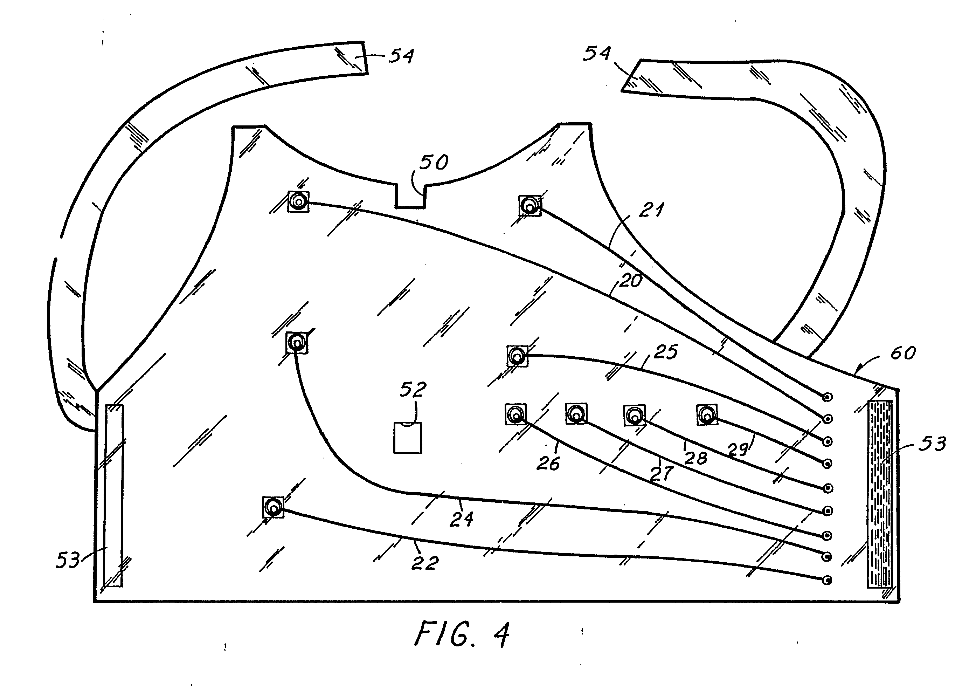

As shown in Figure 4, a bib 60 may be provided with a slot or notch 50 which positions the upper portion of the bib 60 at the clavicular notch. The slot 52 is then utilized to align the bib 60 with the sternum notch. With the alignment along the clavicular notch and sternum notch the electrodes then automatically can be repeatedly placed about the chest when the bib 60 is wrapped around the body since the electrodes maintain the same pattern and location. The bib 60 may be tied with belts 54, 54' or a VELCRO (tradename) fastener 53, 53'.

The bib 60 can be provided in a range of sizes, for example large, medium and small, to accommodate patients of differing stature. The bib can comprise any non-conductive substrate suitable for supporting conductive portions therein. Although the invention has been discussed and illustrated as using lead wires, the invention is not limited to electrically conducting wires, and any technique for incorporating conductive materials in a predetermined pattern into a flexible sheet material can be used. Other modifications can be made without departing from the spirit of the invention, the scope of which is set forth in the following claims.

**Claims**

1. In an apparatus for wearing by a patient about the chest area formed by a flexible substrate having a plurality of electrodes in a predetermined pattern for transmitting electrical impulses from the patient to an electocardiograph, the improvement which comprises a first means for positioning the apparatus at its upper end at the clavicular notch and a second positioning means for positioning the apparatus at the sternum notch, whereby positioning the apparatus at the clavicular notch and at the sternum notch causes positioning of the electrodes about the same position repeatedly.

2. The apparatus set forth in claim 1 wherein each electrode includes
a first end adapted for electrical connection with the skin of the patient; and
a second end adapted for connection with the electrocardiograph device.

3. The apparatus set forth in claim 2 further comprising:
electrically conductive adhesive means on said first ends of said electrodes for securing said substrate to the skin of the patient and assuring electrical contact with the skin.

4. The apparatus of claim 1 wherein said first positioning means comprises a notch and said second positioning comprises an opening.

5. The apparatus set forth in claim 1 wherein said positioning means include one or more contact adhesive patches on a skin contacting surface of said substrate.

6. The apparatus set forth in claim 5 wherein said one or more contact adhesive patches are located on said substrate for overlying bony chest landmarks of the patient.

7. The apparatus set forth in claim 1 further comprising a cable juncture for electrical connection with the electrocardiographic device.

8. The apparatus set forth in claim 1 wherein said plurality of electrodes are mounted on said substrate in predetermined locations corresponding to a standard light lead electrocardiograph configuration.

9. The apparatus set forth in claim 1 wherein said plurality of electrodes are mounted on said substrate in predetermined locations corresponding to a standard twelve lead electrocardiogram configuration.

10. The apparatus of claim 1 further comprising a pattern disposed on said substrate to indicate proper positioning of said substrate on the patient.

11. The apparatus of claim 9 wherein said pattern includes a representation of bony chest landmarks of a patient.

FIG. 1

II

21

20  35  25  10

II

24  27

28

26  29

22

23  30

FIG. 2

10  11

36

35

38

21

FIG. 3

42  43

44  10

20  21

50

25

45  26

24  27  29

28

22  30

23

FIG. 4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,X | EP-A-0 183 410 (R.J. ATHANS) <br> * Abstract; page 4, lines 14-27; page 5, lines 22-30; page 6, lines 1-7,15-20; page 7, lines 7-20,30-35; page 8, lines 8-24; figures 1-4 * | 1,2,4,8 | A 61 B 5/04 |
| A | US-A-3 525 330 (R.E. GREENE) <br> * Abstract; column 2, lines 1-21,32-60; column 3, lines 25-33; column 4, lines 15-27; figures 1,2 * | 1,8,10 | |
| D,A | US-A-4 608 987 (H.E. MILLS) <br> * Abstract; column 3, lines 35-51; column 4, lines 20-25,50-68; column 5, lines 1-12; figure 1 * | 1,2,7,8 ,10,11 | |
| D,A | US-A-4 583 549 (S. MANOLI) <br> * Abstract; column 4, lines 7-21,34-42,49-55; figures 1,5 * | 2,3,5,7 ,8 | |
| A | IBM TECHNICAL DISCLOSURE BULLETIN, vol. 24, no.2, July 1981, pages 1144-1145, Armonk, New York, US; D.C. NEWELL: "ECG standard-patient belt" <br> * Pages 1144,1145 * | 1,2,4,6 -8 | TECHNICAL FIELDS SEARCHED (Int. Cl.4) <br><br> A 61 B |

The present search report has been drawn up for all claims

| Place of search | Date of completion of the search | Examiner |
|---|---|---|
| THE HAGUE | 28-03-1988 | RIEB K.D. |

EPO FORM 1503 03.82 (P0401)